# EUROPEAN PATENT SPECIFICATION

(11) **EP 2 311 503 B1**
(45) Date of publication and mention of the grant of the patent: **09.03.2016**
(21) Application number: 09762600.6
(22) Date of filing: 28.04.2009
(51) Int. Cl.: A61L 9/14, B65D 83/38, B65D 83/26

(54) **AROMA DISPENSER**
AROMASPENDER
DISTRIBUTEUR DE PARFUM

(30) Priority: 13.06.2008 KR 20080055898
(43) Date of publication of application: 20.04.2011
(73) Proprietor: Bae, Sung-Tae, Yuseong-gu Daejeon 305-509 (KR); MAIN CO., LTD., Yuseong-gu Daejeon 305-509 (KR)
(72) Inventor: Bae, Sung-Tae, Daejon 305-509 (KR)
(74) Representative: Isarpatent
(86) International application number: PCT/KR2009/002217
(87) International publication number: WO 2009/151213

(56) References cited:
- WO-A1-2006/084317
- JP-A- 2003 105 827
- KR-A- 20010 088 225
- KR-A- 20010 106 712
- KR-A- 20020 016 384
- KR-B1- 100 473 446
- US-A1- 2007 199 952
- US-B1- 6 296 151

## Description

### [Field of the Invention]

The present invention relates to a fragrance injection device capable of intermittently injecting or spraying a fragrance contained in a spray-type gas container to create a pleasant environment and, more particularly, to a novel fragrance injection device to which only the upper portion of a gas container is fixed for the injection of a fragrance, thereby significantly reducing the overall size of the fragrance injection device while exposing the gas container to the outside. As opposed to the conventional fragrance injection devices designed to fully accommodate a gas container therein without exposing the gas container to the outside, the present invention provides a fragrance injection device in which only the upper portion of a gas container is coupled to the device for the injection of a fragrance in such a fashion that the gas container is exposed to the outside.

### [Background Art]

In recent years, there is available a fragrance therapy by which a fragrance is inhaled through the nose of a patient to facilitate secretion of a neurohormone to thereby prevent an anxiety syndrome, an insomnia and a respiratory disease while achieving psychological stability. In order to perform the fragrance therapy, there have been developed fragrance injection devices capable of injecting or spraying a fragrance contained in a gas container to fill the interior of a room with the fragrance.

The conventional fragrance injection devices are all of the type in which a fragrance is injected or sprayed by pressing the nozzle of a gas container with a solenoid or the like. In the conventional fragrance injection devices, the gas container is fitted into a device body against exposure to the outside.

In other words, the conventional fragrance injection devices have a structure in which the gas container is accommodated within the device body. This makes it impossible to reduce the size of the fragrance injection devices to that of the gas container, meaning that the fragrance injection devices are necessarily greater in size than the gas container.

The increase in the size of the fragrance injection devices results in an increase in the production cost, transportation expense and storage area. In addition, the requirements for non-exposure of the gas container tend to impose constraint on the design of the fragrance injection devices.

US 2007/199952 A1 discloses an automatic discharge device including a housing having a top portion and a base portion, wherein surfaces defining a recess are provided there between for securely holding a container. At least one gear is disposed substantially between the recess and a rear panel of the housing. A drive motor is in association with the at least one gear. An actuator arm is also provided. Activation of the drive motor and the at least one gear provides for movement of the actuator arm between at least one of a pre-actuation position and a discharge position along a path that has a directional component parallel to a longitudinal axis of the recess.

### [Detailed Description of the Invention]

### [Technical Problems]

It is an object of the present invention to provide a fragrance injection device capable of intermittently injecting or spraying a fragrance contained in a spray-type gas container, the fragrance injection device being coupled to the upper portion of the gas container so that the most part of the gas container can be exposed to the outside.

Another object of the present invention is to provide a fragrance injection device of the type that does not accommodate a gas container but can be attached to the upper portion of the gas container. As compared to conventional fragrance injection devices of the type accommodating a gas container therein, the present fragrance injection device is capable of enjoying drastic reduction in volume and improved design, while reducing the production cost, transportation expense and storage area thereof.

### [Solution to the Technical Problems]

According to one aspect of the present invention, there is provided a fragrance injection device according to claim 1.

The fragrance injection device may further comprise a holder member extending from the lower end portion of the body, the holder member having a rear surface flush with the rear surface of the body and a front groove for holding the upper rear surface of the gas container.

In the fragrance injection device, the fixing plate may have an insertion cutout into which the nozzle of the gas container is inserted from the front side in an upright state.

In the fragrance injection device, the cover unit may include an upper cover designed to hide the injector unit and a lower cover configured to surround the upper portion and the nozzle of the gas container, the lower cover including a plurality of locking protrusions engageable with the body and at least one pressing member for pressing the gas container against the body.

In the fragrance injection device, the lower cover may have a through-hole through which the fragrance is injected or sprayed from the nozzle of the gas container.

According to the present disclosure, there is also described a fragrance injection device comprising:
a nozzle head coupled to the upper portion of a gas container containing a fragrance therein, the gas container including an engagement rim formed in the upper portion thereof; and
a solenoid arranged in the nozzle head to control injection of the fragrance from the gas container,
wherein the nozzle head includes hook members pivotally attached to the opposite side surfaces thereof for holding the engagement rim of the gas container, a spring for biasing the hook members into engagement with the engagement rim of the gas container and buttons provided in the hook members for, when pressed, causing the hook members to be disengaged from the engagement rim of the gas container against the biasing force of the spring.

### [Advantageous Effects]

The present fragrance injection device does not accommodate a gas container therein but can be attached to the upper portion of the gas container. As compared to conventional fragrance injection devices of the type accommodating a gas container therein, it is possible for the present fragrance injection device to enjoy drastic reduction in volume with a compact design, while reducing the production cost, transportation expense and storage area thereof.

### [Brief Description of the Drawings]

Fig. 1 is a perspective view showing a fragrance injection device in accordance with one embodiment of the present invention, with the lower cover thereof kept in an open state.
Fig. 2 is an exploded perspective view of the present fragrance injection device.
Fig. 3 is a front view of the present fragrance injection device.
Fig. 4 is a rear view of the present fragrance injection device.
Fig. 5 is an exploded side view of the present fragrance injection device.
Fig. 6 is an exploded bottom view of the present fragrance injection device.
Fig. 7 is an exploded rear perspective view of the present fragrance injection device.
Fig. 8 is a section view showing a fragrance injection device in accordance with the present disclosure, in which view no gas container is coupled to a nozzle head.
Fig. 9 is a section view similar to Fig. 8, showing the state that a gas container is coupled to a nozzle head.
Fig. 10 is a front view showing a fragrance injection device in accordance with the present disclosure, with a gas container coupled thereto.
Fig. 11 is a perspective view of the fragrance injection device shown in Fig. 10.

### [Best Mode for Carrying out the Invention]

The present invention is directed to a fragrance injection device for periodically pressing or opening the nozzle of a gas container filled with a fragrance so that the fragrance can be injected or sprayed into the interior of a room. The fragrance injection device is coupled to the upper portion of the gas container, the most part of which remains exposed to the outside. This makes it possible to reduce the size of the fragrance injection device while simplifying the design thereof.

In the present fragrance injection device, only the upper portion of the gas container having a nozzle is coupled to the fragrance injection device, with the gas container exposed to the outside for its most part. The fragrance injection device is designed to inject or spray a fragrance by pressing or opening the nozzle of the gas container. The fragrance injection device includes a body, a coupler unit for coupling the gas container to the body through the use of an engagement rim formed in the upper portion of the gas container, a cover unit for preventing the gas container from being inadvertently removed from the body, an injector unit for causing the fragrance to be periodically injected from the nozzle of the gas container, and a control unit supplied with electric power from a battery for controlling the operation of the injector unit.

More specifically, the fragrance injection device is provided with the coupler unit integrally formed with the body. The engagement rim of the gas container is fitted to the coupler unit from the front side against removal in the vertical direction. The cover unit is configured to cover the front surface of the body and to press the gas container against the coupler unit. The injector unit for pressing or opening the nozzle of the gas container and the control unit for operating the injector unit are arranged in the inner upper portion of the body. The battery is provided inside the body to supply electric power to the control unit.

The fragrance injection device further includes a holder member extending downwards from the rear lower end of the body. The holder member serves to prevent the lower portion of the body from being pushed rearwards when the nozzle of the gas container is pressed by the injector unit. The holder member comes into contact with a wall surface in case where the fragrance injection device is hung on the wall surface. This helps prevent the lower portion of the body from being moved rearwards by the force applied to the nozzle of the gas container.

In order to couple the gas container to the fragrance injection device, the cover unit is opened to expose the interior of the body. Then, the engagement rim of the gas container is fitted to the coupler unit. In this state, the cover unit is closed to hold the gas container against any back-and-forth movement, thereby terminating the container coupling process. The holder member extending downwards from the body remains in contact with the wall surface when the fragrance injection device is hung on the wall surface.

In this regard, the body is designed to accommodate the battery, the injector unit and the control unit therein. The injector unit may be designed to press the nozzle of the gas container by converting the rotational movement of an electric motor to the vertical linear movement of a pressing member through the use of a gear train. Alternatively, the injector unit may employ a solenoid for opening the mouth of the nozzle. The injector unit and the control unit are well-known in the art and have been disclosed by the present applicant.

Hereinafter, the fragrance injection device in accordance with one embodiment of the present invention will be described in detail with reference to the accompanying drawings.

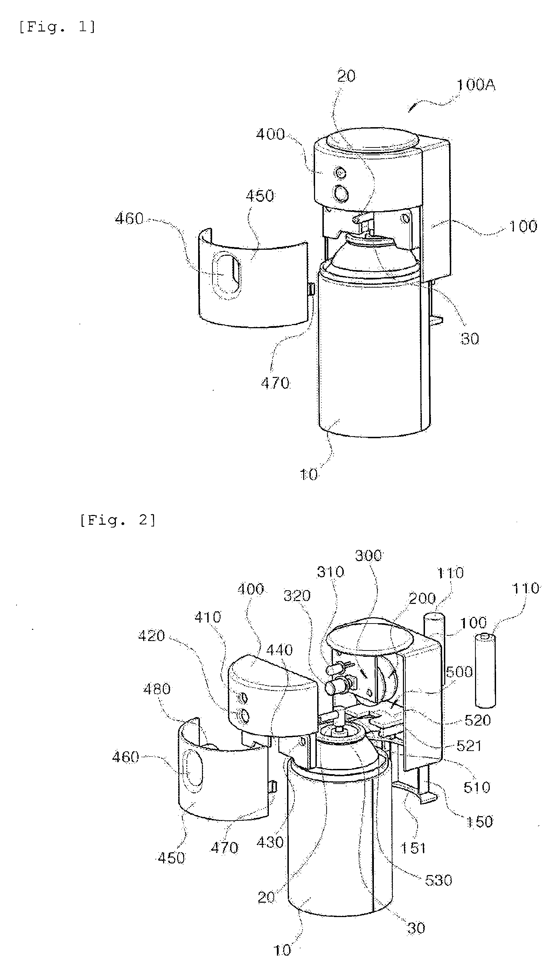

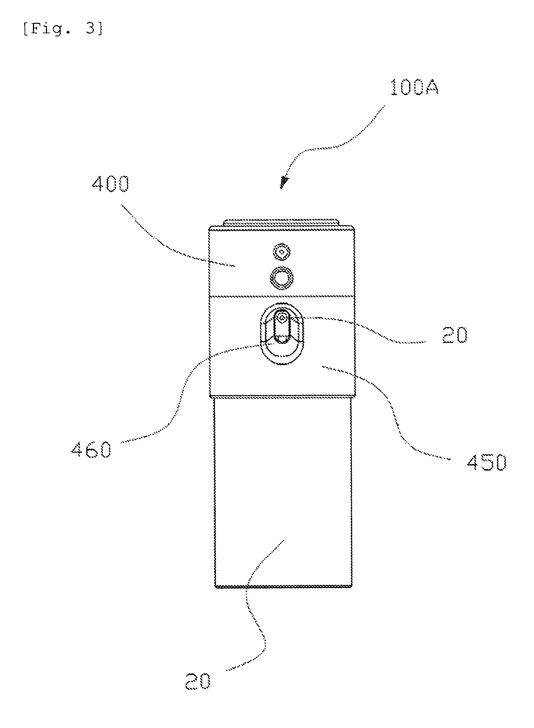

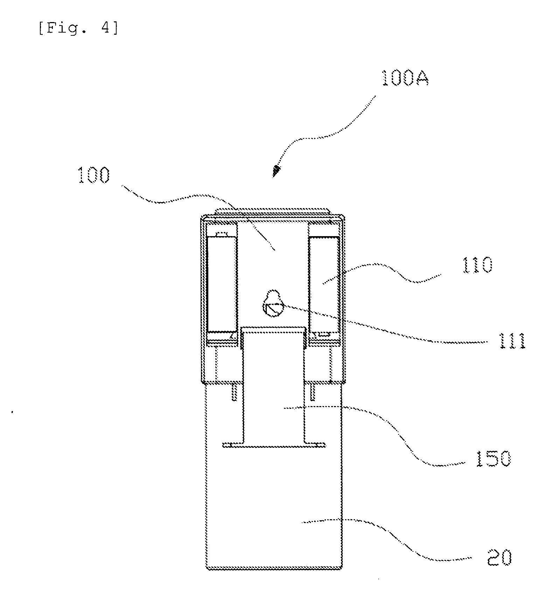

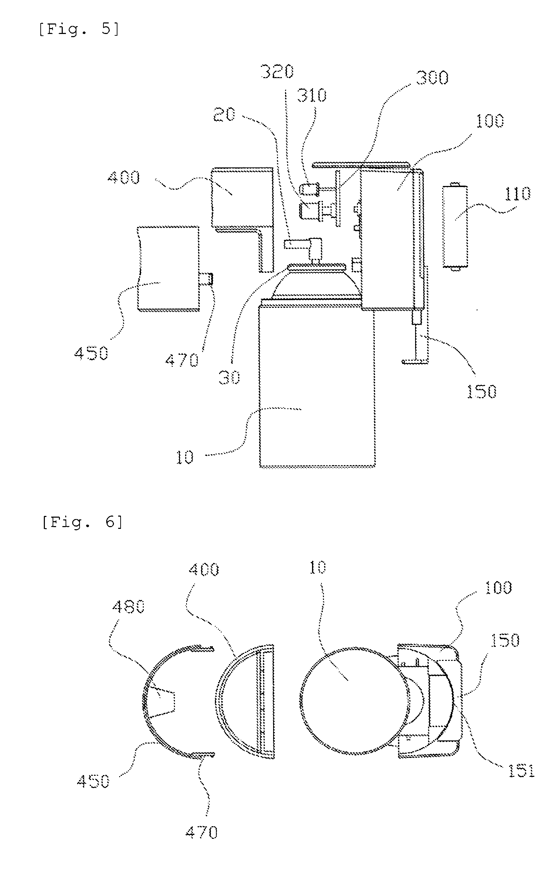

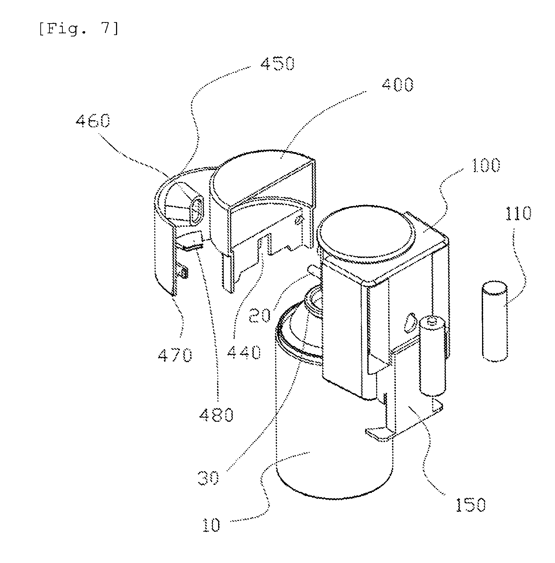

Referring to Figs. 1 through 7, the present fragrance injection device 100A is coupled to the upper portion of a gas container 10 well-known in the art, with the most part of the fragrance injection device 100A exposed to the outside. As compared to conventional fragrance injection devices of the type accommodating a gas container therein, the present fragrance injection device is capable of enjoying drastic reduction in volume and having a simple design.

The fragrance injection device 100A includes a body 100, an injector unit 200, a control unit 300 and a coupler unit 500. The body 100 is provided with a holder member 150 extending downwards from the lower end of the body 100. Batteries 110 can be removably inserted into the body 100 from the rear side of the latter. A gas container 10 containing a liquid fragrance and a carrier gas is removably coupled to the coupler unit 500. The gas container 10 is provided with a nozzle 20, through which the fragrance can be injected or sprayed from the gas container 10, and an engagement rim 30. The fragrance injection device 100A further includes a lower cover 450 for covering the front surface of the body 100 to prevent inadvertent removal of the gas container 10.

The coupler unit 500 for holding the gas container 10 is installed in the body 100. The coupler unit 500 includes a fixing plate 520 integrally formed with the body 100 and a generally-semicircular support flange 510 arranged below the fixing plate 520. The fixing plate 520 has an insertion cutout 521 into which the nozzle 20 of the gas container 10 can be inserted by a user. A coupling groove 530 is defined between the fixing plate 520 and the support flange 510 so that the engagement rim 30 of the gas container 10 can be fitted into the coupling groove 530.

In this regard, the support flange 510 needs to have an inner edge conforming to the shape of the gas container 10. The fixing plate 520 has the strength great enough to hold the gas container 10 inserted into the coupling groove 530. The insertion groove 521 of the fixing plate 520 is shaped so that the nozzle 20 of the gas container 10 can be inserted into the insertion groove 521 from the front side.

The body 100 is opened at its lower end so that the engagement rim 30 of the gas container 10 can be fitted into the coupling groove 530 between the support flange 510 and the fixing plate 520. The injector unit 200 and the control unit 300 are arranged above the coupler unit 500. The lower cover 450 is removably attached to the front surface of the body 100.

The injector unit 200 serves to, when operated, press the nozzle 20 of the gas container 10 to inject or spray the fragrance contained in the gas container 10. As is well-known in the art, the injector unit 200 includes an electric motor (not shown) and a gear train (not shown) for converting the rotational movement of the electric motor to the vertical linear movement needed to press the nozzle 20 of the gas container 10. The control unit 300 serves to control the operation of the injector unit 200 and the operation time of the latter. An indicator lamp 310 and a switch 320 are arranged in front of the control unit 300. The structures and functions of the control unit 300, the indicator lamp 310 and the switch 320 are well-known in the art and therefore will not be described in more detail.

An upper cover 400 for covering the control unit 300 is fixed to the front surface of the body 100 by tightening screws into thread holes 430. The upper cover 400 has a pair of holes through which to expose the indicator lamp 310 and the switch 320 and an insertion cutout 440 into which the nozzle 20 of the gas container 10 is inserted from the lower side. The upper cover 400 remains attached to the body 100 unless there is a need to repair the fragrance injection device 100A.

The lower portion of the upper cover 400 and the front surface of the body 100 are covered by the lower cover 450. The lower cover 450 has a front hole 460 into which the tip end of the nozzle 20 is inserted, a pair of locking protrusions 470 formed at the lateral rear edges thereof, and a pair of pressing members 480 formed on the rear surface thereof for pressing the engagement rim 30 of the gas container 10. When fixing the lower cover 450 to the front surface of the body 100 by the locking protrusions 470, the pressing members 480 press the engagement rim 30 of the gas container 10 against movement. The locking protrusions 470 are removed from the body 100 if the lower cover 450 is pressed inwards and then pulled forwards.

The body 100 has a hanger hole 111 formed on the rear surface thereof and used in hanging the fragrance injection device 100A on a wall surface. Batteries 110 are inserted into the rear side spaces of the body 100 to supply electric power to the control unit 300 and the injector unit 200. The rear side spaces are closed by lids which can be removed for replacement of the batteries 110.

The body 100 is provided with a holder member 150 extending downwards from the lower end of the body 100. The rear surface of the holder member 150 is flush with that of the body 100 so that the holder member 150 and the body 100 can make simultaneous contact with the wall surface when the fragrance injection device 100A is hung on the wall surface. The holder member 150 has a semicircular retainer groove 151 conforming to the shape of the gas container 10. This helps prevent the gas container 10 from being moved backwards when in use.

In order to attach the gas container 10 to the fragrance injection device 100A, the lower cover 450 needs to be removed from the body 100. To this end, the lateral end portions of the lower cover 450 are pressed toward each other to have the locking protrusions 470 removed from the body 100. Then, the lower cover 450 is pulled forwards away from the body 100, in which state the coupler unit 500 and the holder member 150 can be seen from the front side.

In this state, the gas container 10 is pushed toward the body 100 so that the nozzle 20 can be inserted into the insertion cutout 521 of the fixing plate 520 with the tip end of the nozzle 20 faced forwards. As a result, the engagement rim 30 of the gas container 10 is fitted into the coupling groove 530 defined between the support flange 510 and the fixing plate 520. Thus, the gas container 10 is coupled to the body 100 of the fragrance injection device 100A.

At this time, the support flange 510 of the coupler unit 500 serves to support the lower surface of the engagement rim 30 of the gas container 10, while the fixing plate 520 functions to press the upper surface of the engagement rim 30. This helps prevent the gas container 10 from moving upwards or downwards.

Once the gas container 10 is coupled with the fragrance injection device 100A, the rear surface thereof is held by the retainer groove 151 of the holder member 150 extending downwards from the lower end of the body 100.

Thereafter, the lower cover 450 is fixed to the front surface of the body 100 in such a fashion that the locking protrusions 470 can come into engagement with the body 100. The pressing members 480 of the lower cover 450 serve to press the gas container 10 against body 100 and the holder member 150, thereby preventing inadvertent movement of the gas container 10. At this time, the nozzle 20 of the gas container 10 is oriented to face toward the hole 460 of the lower cover 450 so that the fragrance injected or sprayed from the nozzle 20 can be dispensed into the air through the hole 460.

If necessary, the gas container 10 can be replaced with a new one by removing the lower cover 450, exchanging the old container with a new one and reattaching the lower cover 450 to the body 100.

The fragrance injection device 100A having the gas container 10 coupled thereto can be hung on a wall surface though the use of the hanger hole 111 formed on the rear surface of the body 100. In this state, the rear surfaces of the body 100 and the holder member 150 come into contact with the wall surface. If the switch 320 is turned on in this state, the control unit 300 operates the injector unit 200 with the electric power supplied from the batteries 110. In response, the injector unit 200 periodically presses the nozzle 20 of the gas container 10 so that the fragrance can be injected or sprayed from the nozzle 20. The period in which the injector unit 200 is operated by the control unit 300 can be arbitrarily set by means of a timer. The operating condition of the fragrance injection device 100A is notified to a user by the indicator lamp 310.

When the nozzle 20 is pressed by the injector unit 200, the lower extension of the body 100 may be applied with a backwardly-acting force. This force can be borne by the holder member 150, thereby preventing the lower extension of the body 100 from moving backwards.

As opposed to conventional fragrance injection devices in which a gas container is fully accommodated within a device body, the present fragrance injection device 100A is coupled to the upper portion of the gas container 10 in such a manner that the gas container 10 is exposed to the outside for the most part. The present fragrance injection device 100A does not substantially differ from the conventional ones in that the nozzle 20 of the gas container 10 is pressed by the injector unit 200 to inject or spray the fragrance. The present fragrance injection device 100A configured as above is capable of enjoying drastic reduction in volume and improved design, while reducing the production cost, transportation expense and storage area thereof.

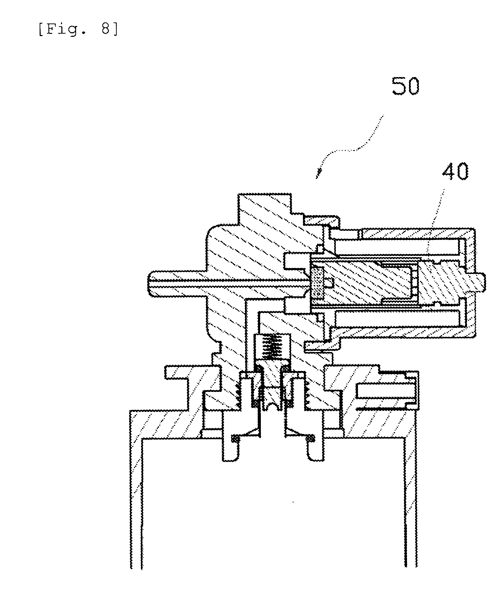

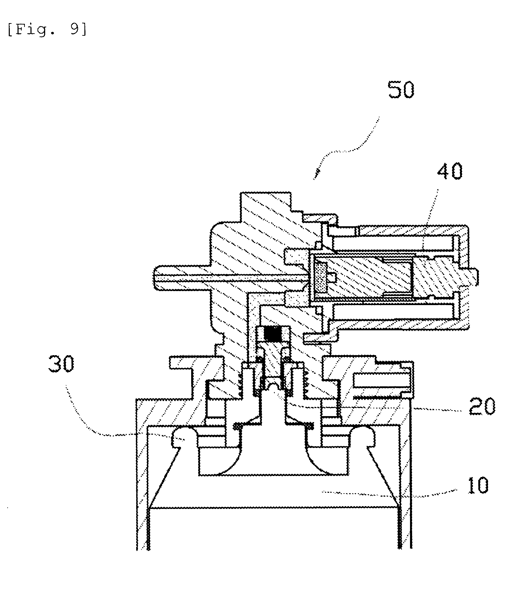

Referring to Figs. 8 and 9, there is shown another fragrance injection device. This fragrance injection device differs from the fragrance injection device 100A of the preceding embodiment in that a solenoid 40 is used to open the nozzle 20. In this fragrance injection device, the gas container 10 is mounted to a nozzle head 50. The mouth of the nozzle 20 is normally closed by the solenoid 40. Upon energizing the solenoid 40, the mouth of the nozzle 20 is opened so that the fragrance can be injected or sprayed from the nozzle 20.

In case where a gear mechanism is used as the injector unit 200, it is necessary to employ a constant-volume valve in which a specified amount of fragrance is injected upon pressing the nozzle 20 at one time. The fragrance is not injected while the nozzle 20 is not pressed. In case of the fragrance injection device using the solenoid 40, the fragrance continues to be injected upon mounting the gas container 10 to the nozzle head 50. The injection of fragrance is stopped when the solenoid 40 is energized.

In the injector unit 200 in which the nozzle 20 is pressed by the gear mechanism, the gas container 10 is fixed in place by coupling the neck of the gas container 10 to the fragrance injection device in a key-coupling method. This does not differ from the foregoing embodiment.

In the injector unit 200 of the type using the solenoid 40, however, it is of paramount importance that the gas container 10 is securely fixed to the nozzle head 50. Since a gas pressure of about 7 kg/cm² acts within the gas container 10, a measure should be taken to keep the gas container 10 in place and to prevent leakage of the fragrance when the gas container 10 and the nozzle head 50 are coupled together.

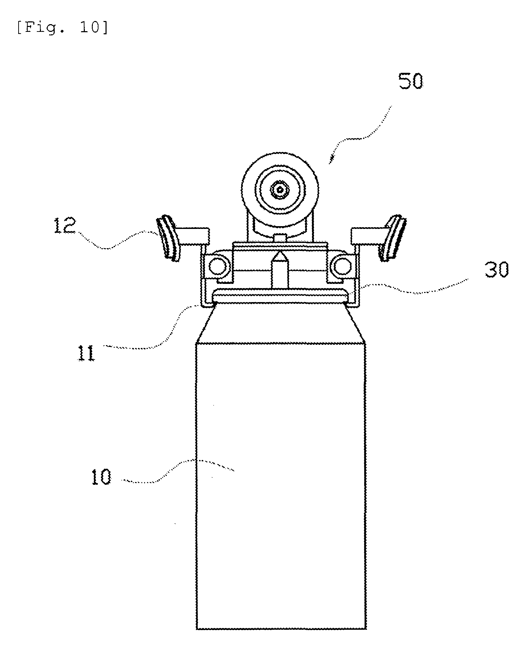

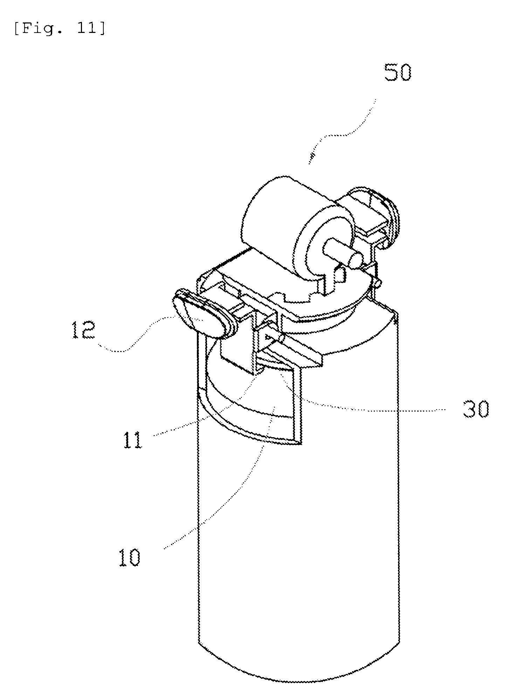

Referring to Figs. 10 and 11, there is shown a further fragrance injection device. In this fragrance injection device, the nozzle head 50 includes a pair of hook members 11 for holding the engagement rim 30 of the gas container 10 and a pair of buttons 12 provided at the top ends of the hook members 11. The hook members 11 are pivotally fixed to the neck of the nozzle head 50 and the lower ends of the hook members 11 are biased toward each other by torsion springs (not shown). If the buttons 12 are pressed with the fingers of a user, the hook members 11 are spread apart from each other. In this state, the gas container 10 is moved upwards and coupled to the nozzle head 50. If the buttons 12 are released, the lower ends of the hook members 11 are moved toward each other by the torsion springs, thus holding the engagement rim 30 of the gas container 10. This makes it possible to securely fix the gas container 10 to the nozzle head 50.

### [Industrial Applicability]

The present fragrance injection device can be used in combination with a gas container containing a fragrance under pressure. The fragrance injection device is periodically operated to open the gas container and to inject or spray the fragrance contained in the gas container.

## Claims

1. A fragrance injection device (100A) usable in combination with a gas container (10) containing a fragrance therein, the gas container (10) including an upper portion, a nozzle (20) and an engagement rim (30) formed in the upper portion, comprising:
a body (100) designed to cover the upper portion of the gas container (10), the body (100) including a front surface, a rear surface and a lower end portion;
a coupler unit (500) provided in the body (100) for coupling the upper portion of the gas container (10) and the body (100) together;
an injector unit (200) arranged inside the body (100) for, when operated, opening the nozzle (20) of the gas container (10) to periodically inject or spray the fragrance contained in the gas container (10);
a control unit (300) arranged inside the body (100) for controlling the operation of the injector unit (200); and
a cover unit removably fixed to the body (100) for covering the front surface of the body (100), the cover unit including an upper cover (400) designed to hide the injector unit (200) and a lower cover (450) configured to surround the upper portion and the nozzle (20) of the gas container (10), the lower cover (450) including a plurality of locking protrusions (470) engageable with the body (100) and at least one pressing member (480) for pressing the gas container (10) against the body (100), wherein the coupler unit (500) includes a fixing plate (520) integrally formed with the body (100) and a generally-semicircular support flange (510) arranged below the fixing plate (520), the fixing plate (520) having an insertion cutout (521) into which the nozzle (20) of the gas container (10) is insertable, and the coupler unit (500) further comprising a coupling groove (530) being defined between the fixing plate (520) and the support flange (510) so that the engagement rim (30) of the gas container (10) is fittable into the coupling groove (530).

2. The fragrance injection device as recited in claim 1, further comprising a holder member (150) extending from the lower end portion of the body (100), the holder member (150) having a rear surface flush with the rear surface of the body (100) and a front groove for holding the upper rear surface of the gas container (10).

3. The fragrance injection device as recited in claim 1, wherein the fixing plate (520) has an insertion cutout (521) into which the nozzle (20) of the gas container (10) is inserted from the front side in an upright state.

4. The fragrance injection device as recited in claim 3, wherein the lower cover (450) has a through-hole through which the fragrance is injected or sprayed from the nozzle (20) of the gas container (10).

## Patentansprüche

1. Duftstoffinjizierungsvorrichtung (100A), die in Kombination mit einem Gasbehälter (10) verwendet werden kann, der einen Duftstoff enthält, wobei der Gasbehälter (10) einen oberen Abschnitt, eine Düse (20) und einen Eingriffnahmerand (30), der in dem oberen Abschnitt ausgebildet ist, umfasst, und die Folgendes umfasst:
einen Körper (100), der dafür ausgelegt ist, den oberen Abschnitt des Gasbehälters (10) zu bedecken, wobei der Körper (100) eine Vorderfläche, eine Rückfläche und einen unteren Endabschnitt umfasst;
eine Kopplereinheit (500), die in dem Körper (100) angeordnet ist, um den oberen Abschnitt des Gasbehälters (10) und den Körper (100) miteinander zu koppeln;
eine Injektoreinheit (200), die im Inneren des Körpers (100) angeordnet ist, um, wenn sie betätigt wird, die Düse (20) des Gasbehälters (10) zu öffnen, um den in den Gasbehälter (10) enthaltenen Duftstoff periodisch zu injizieren oder zu versprühen;
eine Steuereinheit (300), die im Inneren des Körpers (100) angeordnet ist, um die Operation der Injektoreinheit (200) zu steuern; und
eine Abdeckeinheit, die abnehmbar an dem Körper (100) befestigt ist, um die Vorderfläche des Körpers (100) abzudecken, wobei die Abdeckeinheit eine obere Abdeckung (400) umfasst, die dafür ausgelegt ist, die Injektoreinheit (200) zu verbergen, und eine untere Abdeckung (450) umfasst, die dafür ausgebildet ist, den oberen Abschnitt und die Düse (20) des Gasbehälters (10) zu umgeben, wobei die untere Abdeckung (450) mehrere Verriegelungsvorsprünge (470) umfasst, die mit dem Körper (100) in Eingriff gebracht werden können, und mindestens ein Presselement (480) umfasst, um den Gasbehälter (10) gegen den Körper (100) zu pressen, wobei die Kopplereinheit (500) eine Befestigungsplatte (520) umfasst, die einstückig mit dem Körper (100) ausgebildet ist, und einen allgemein halbkreisförmigen Stützflansch (510) umfasst, der unterhalb der Befestigungsplatte (520) angeordnet ist, wobei die Befestigungsplatte (520) einen Einschubausschnitt (521) aufweist, in den die Düse (20) des Gasbehälters (10) eingeschoben werden kann, und die Kopplereinheit (500) des Weiteren eine Kopplungsnut (530) umfasst, die zwischen der Befestigungsplatte (520) und dem Stützflansch (510) so definiert ist, dass der Eingriffnahmerand (30) des Gasbehälters (10) in die Kopplungsnut (530) eingepasst werden kann.

2. Duftstoffinjizierungsvorrichtung nach Anspruch 1, die des Weiteren ein Halteelement (150) umfasst, das sich von dem unteren Endabschnitt des Körpers (100) erstreckt, wobei das Halteelement (150) eine Rückfläche aufweist, die bündig mit der Rückfläche des Körpers (100) abschließt, und eine vordere Nut aufweist, um die obere Rückfläche des Gasbehälters (10) zu halten.

3. Duftstoffinjizierungsvorrichtung nach Anspruch 1, wobei die Befestigungsplatte (520) einen Einschubausschnitt (521) hat, in den die Düse (20) des Gasbehälters (10) von der Vorderseite her in einem aufrechten Zustand eingeschoben wird.

4. Duftstoffinjizierungsvorrichtung nach Anspruch 3, wobei die untere Abdeckung (450) ein Durchgangsloch hat, durch das der Duftstoff aus der Düse (20) des Gasbehälters (10) injiziert oder versprüht wird.

## Revendications

1. Dispositif d'injection de fragrance (100A) utilisable en combinaison avec un conteneur de gaz (10) contenant une fragrance à l'intérieur, le conteneur de gaz (10) comprenant une partie supérieure, une buse (20) et un bord de prise (30) réalisé dans la partie supérieure, comprenant :
un corps (100) conçu pour couvrir la partie supérieure du conteneur de gaz (10), le corps (100) comprenant une surface avant, une surface arrière et une partie d'extrémité inférieure ;
une unité de coupleur (500) prévue dans le corps (100) pour coupler la partie supérieure du conteneur de gaz (10) et le corps (100) l'un à l'autre ;
une unité d'injecteur (200) agencée à l'intérieur du corps (100) pour, lorsqu'elle fonctionne, ouvrir la buse (20) du conteneur de gaz (10) afin d'injecter ou de pulvériser périodiquement la fragrance contenue dans le conteneur de gaz (10) ;
une unité de commande (300) agencée à l'intérieur du corps (100) pour commander le fonctionnement de l'unité d'injecteur (200) ; et
une unité de couvercle fixée de manière amovible au corps (100) pour couvrir la surface avant du corps (100), l'unité de couvercle comprenant un couvercle supérieur (400) conçu pour cacher l'unité d'injecteur (200) et un couvercle inférieur (450) configuré pour entourer la partie supérieure et la buse (20) du conteneur de gaz (10), le couvercle inférieur (450) comprenant une pluralité de saillies de verrouillage (470) pouvant venir en prise avec le corps (100) et au moins un élément de pression (480) pour presser le conteneur de gaz (10) contre le corps (100), dans lequel l'unité de coupleur (500) comprend une plaque de fixation (520) formée d'un seul tenant avec le corps (100) et une bride de support globalement semi-circulaire (510) agencée en dessous de la plaque de fixation (520), la plaque de fixation (520) présentant une découpe d'insertion (521) dans laquelle la buse (20) du conteneur de gaz (10) est insérable, et l'unité de coupleur (500) comprenant en outre une rainure de couplage (530) définie entre la plaque de fixation (520) et la bride de support (510) de telle sorte que le bord de prise 30) du conteneur de gaz (10) peut être mis en place dans la rainure de couplage (530).

2. Dispositif d'injection de fragrance selon la revendication 1, comprenant en outre un élément de maintien (150) s'étendant depuis la partie d'extrémité inférieure du corps (100), l'élément de maintien (150) présentant une surface arrière dans l'alignement de la surface arrière du corps (100) et une rainure avant pour maintenir la surface arrière supérieure du conteneur de gaz (10).

3. Dispositif d'injection de fragrance selon la revendication 1, dans lequel la plaque de fixation (520) présente une découpe d'insertion (521) dans laquelle la buse (20) du conteneur de gaz (10) est insérée depuis le côté avant dans une position debout.

4. Dispositif d'injection de fragrance selon la revendication 3, dans lequel le couvercle inférieur (450) présente un trou traversant à travers lequel la fragrance est injectée ou pulvérisée depuis la buse (20) du conteneur de gaz (10).
